# EUROPEAN PATENT APPLICATION

(11) **EP 3 756 711 A1**
(43) Date of publication of application: **30.12.2020**
(21) Application number: 19182370.7
(22) Date of filing: 25.06.2019
(51) Int. Cl.: A61M 15/00, B05D 1/00, B05D 3/10, B05D 7/14, B65D 83/14, B65D 83/54, C23C 14/02, C23C 14/12, C23C 16/02, C23C 16/44, C23C 16/50, C23G 5/00, C23G 5/02, C23G 5/06, B08B 7/00, C11D 11/00

(54) **METHOD OF PLASMA CLEANING AND COATING OF MDI CANS**

(71) Applicant: Presspart GmbH & Co. KG, 34431 Marsberg (DE)
(72) Inventor: ZWICK, Martin, 34431 Marsberg (DE); BACKHAUS, Stefan, 34431 Marsberg (DE); SCHLENKE, Andreas, 34431 Marsberg (DE); RAMSPOTT, Janis, 34431 Marsberg (DE); GIEBEL, Andreas, 33098 Paderborn (DE); SCHULTE, Christoph, 33098 Paderborn (DE)
(74) Representative: Uexküll & Stolberg

(57) **Abstract**

A method of cleaning and coating at least one coating of a metered dose inhaler comprises the step of cleaning the at least one surface of the container with a cleaning solvent, an aqueous cleaning solution on alcohol based cleaning solution. The method further comprises the step of subjecting the cleaned at least one surface to an atmospheric plasma treatment. In a subsequent step of the method the plasma treat at least one surface is subjected to a coating composition containing a flourolpolymere resin or to a plasma coating treatment.

## Description

### FIELD OF THE INVENTION

The present invention relates to the production of metered dose inhaler containers (MDI-containers), in particular to the cleaning and coating of MDI-containers. The invention also relates to a processing line for cleaning and coating of MDI-containers.

### BACKGROUND OF THE INVENTION

Drugs for treating respiratory and nasal disorders are frequently administered in aerosol formulation through the mouth or nose of a patient. One widely used method for dispensing such aerosol drug formulations involves preparing a suspension of a solution formulation of the drug and of a liquefied gas known as a propellant. The suspension is stored in a sealed container (or canister) capable of withstanding the pressure required to maintain the propellant as a liquid. The formulation may then be dispersed by activating a dose-metering valve fixed to the container.

Metered dose inhalers (MDI) are typically used to dispense such drug formulations. The container is inserted into the MDI such that the metering valve is received in a nozzle block located at a bottom portion of the inhaler. The metering valve of the container is designed to consistently release a fixed predetermined mass of the aerosol drug formulation upon activation of the MDI, in particular upon depression of the container against the nozzle block. As the formulation is forced from the container through the dose-metering valve and the nozzle block by the high vapor pressure of the propellant, the suspension rapidly exits the nozzle block via a nozzle. Upon exiting the nozzle block the suspension rapidly vaporizes leaving a fast moving plume of very fine particles of the drug formulation. This plume of particles is directed into the mouth of the patient via a mouthpiece of the MDI and is inhaled by the patient into his/her lungs.

Patients often rely on medication delivered by MDIs for rapid treatment of respiratory disorders, including for example asthma and chronic obstructive pulmonary disease (COPD). Therefore, it is essential that the prescribed dose of aerosol medication delivered to the patient consistently meet the specifications claimed by the manufacturer. That is, every dose in the container must be the same within close tolerances.

In some instances, the aerosol drug formulation may tend to adhere to and/or chemically react with the inner surfaces of the MDI, including the canister, metering valve and cap. This can lead to the patient receiving significantly less or more than the prescribed amount of drug upon each activation of the MDI. Thus, it is often desirable to coat the inner surface of a container, with a coating material that prevents the drug formulation from maturing to or reacting with the inner surfaces of the MDI.

Fluorine-containing polymers have been widely used for the coating of an inner surface of MDI container. For example US 6,596,260 relates to an aerosol container for pharmaceutically active aerosols that are to be administered in predetermined amounts and that are supplied in the container in the form of a suspension. The inner wall of the container is coated with polytetrafluoroethylene, also widely known by the name Teflon.

In order to provide a proper adherence of the coating to the MDI container cleaning of the inner surface of the container prior to its coating is essential. Conventionally, the majority of MDI-containers have been solvent-cleaned with a proprietary commercially supplied mixture of hydrocarbons and emulsifiers. While these remove most of the hydrocarbon lubricants used for the production of the container, in particular via deep drawing or impact extrusion, the resulting surface contains residual oils and fatty acids that prevent wetting with coatings. Thus, the MDI containers typically have to be heated above 200°C in an oven to burn off residual hydrocarbons and have to be cooled thereafter. As the containers have to be warmed and cooled thoroughly the heating and cooling takes a considerable time.

It is an object of the present invention to provide a method of cleaning and coating of MDI container with an increased production rate but which at the same time allows proper adherence of a coating composition to at least one surface of the MDI-container.

### SUMMARY OF THE INVENTION

The object is achieved with the method of cleaning and coating as defined in present claim 1. Preferred embodiments are set out in the dependent claims.

According to the invention the provided method is suitable for cleaning and coating at least one surface of a metered dose inhaler container, the method comprising the steps of:
a) cleaning the at least one surface of the container with a cleaning solvent, an aqueous cleaning solution or an alcohol based cleaning solution;
b) subjecting the cleaned at least one surface to an atmospheric plasma treatment;
c) subjecting the plasma treated at least one surface to a coating composition containing a fluoropolymer resin or to a plasma coating treatment.

Optionally, in case the plasma treated at least one surface is subjected to a coating composition containing a fluoropolymer resin the following further method step may be carried out:
d) subjecting the coated at least one surface to a cross-linking step to provide an adherent continuous coating on the at least one surface of the container.

However, in case the plasma treated at least one surface is subjected to a plasma coating treatment no cross-linking-step is needed.

In connection with the present invention, it has been found that atmospheric plasma is suitable to clean and/or activate the at least one surface of the MDI container prior to being coated. In connecting with the present invention the term "activate" is understood as increasing the surface tension of a surface. Activating a surface improves wettability of the surface and the adherence of a later coating thereon. Moreover, in connection with the present invention the term "atmospheric plasma" is understood as plasma having the pressure of the surrounding atmosphere, i.e. around 101,325 kPa at sea level and at 0°C. Thus, using atmospheric plasma there is no need for a reaction chamber providing an increased or decreased pressure level compared to the surrounding atmosphere.

The use of atmospheric plasma is for example described in EP 0 761 415 B1 to which reference is made in its entirety. This document relates to the pre-treatment of surfaces of thin work pieces made from plastic, in particular plastic foils. However, metal surfaces may also be treated in order to remove silicon residues.

In connection with the present invention it has been found that the use of atmospheric plasma increases wettabilty of the at least one surface of the MDI container to be treated. Compared to a conventional cleaning and coating process in which a cleaning solvent is used and the MDI container has to be heated and cooled thereafter to remove the residual hydrocarbons thereon, the thermal treatment steps, i.e. heating and cooling in a separate oven, can be omitted. Compared to such a conventional method of cleaning and coating, the time needed to produce each MDI container by use of the method according to the present invention was decreased by a factor ranging from 1,5 to 2, in particular around 1,8. Assuming that a conventional method would allow a production of 90 cans per minute the method according the present invention allows a production of 160 cans per minute.

Moreover, the use of atmospheric plasma for cleaning the at least one surface of the MDI container allows the use of multiple alternative cleaning solutions such as a cleaning solvent, an aqueous cleaning solution or an alcohol based cleaning solution. Using atmospheric plasma in a cleaning step allows removement of any residues left on the at least one surface from the production process of the MDI container, in particular deep drawing or impact extrusion, and/or from the cleaning solutions used. Thus, by use of atmospheric plasma the at least one surface is properly prepared for coating regarding its degree of purity and its degree of wettabilty with a coating composition. Proper adherence of the coating to the at least one surface is provided and a reaction of the solution contained in the container and the at least one surface of the MDI container is omitted.

Furthermore, the use of atmospheric plasma allows the coating of the at least one surface with a coating composition containing a fluoropolymer resin or by use of a plasma coating treatment. The coating composition and the plasma coating treatment can be used as alternatives. A method and corresponding device for a plasma coating treatment is for example disclosed in document EP 2 539 082 B1 to which reference is made in its entirety. According to EP 2 539 082 B1 an inner surface of a medicament body is coated with a plasma polymerised polymer coating. Optionally, the plasma treatment is a low pressure plasma treatment.

In an embodiment of the invention the cleaning solvent comprises chlorinated hydrocarbons. Preferably, the cleaning solvent is tetrachlorethene (PER). The use of PER is advantageous as it has a high degreasing capability.

In another embodiment of the invention the aqueous cleaning solution comprises an anionic surfactant and emulsifier. The surfactant and the emulsifier promote emulsification with water. Suitable anionic surfactants include sodium dodecylbenzene sulfonate, alkyl phosphate, alkyl sulfonate, alkyl benzenesulfonate, and sodium di(2-ethylhexyl) sulfosuccinate. Preferably, the anionic surfactant is dodecylbenzene sulfonate. The anionic surfactant is used to penetrate the oil film on the preferably deep drawn or impact extruded cans and to emulsify and disperse the oil in the cleaning bath.

In another embodiment of the invention, the coating composition is solvent based or aqueous based. Both coating compositions can be used as alternatives and have shown proper adherence to the at least on surface of the MDI container.

In an embodiment of the invention the solvent based coating composition comprises a solvent chosen from the group consisting of: n-methyl-2-pyrrolidone (NMP), n-butyl-2-pyrrolidone (NBP) and methylisobutylketone (MIBK).

In another embodiment the aqueous based coating composition comprises 20-40 wt% of the fluoropolymer resin with one or more alcohols as wetting agents and balance water. Examples of alcohols usable as wetting agents in the practice of the present invention include but are not limited to, n-butyl alcohol (about 2-10 wt%), 2-butoxyethanol (about 2-10 wt%), and/or 2-(dimethylamino)ethanol (about 1-5 wt%), and amyl alcohol, with water as the balance. The concentration of the fluoropolymer resin is adjusted for optimum spray efficiency, surface finish, and thermal desorption, with the optimum between 22 and 27 wt%.

In an embodiment the fluoropolymer resin is chosen from the group consisting of: polytetrafluoroetylene (PTFE), fluorinated ethylene propylene (FEP) and perflouroalkoxy polymers (PFA).

Preferably, the atmospheric plasma treatment includes generating a concentrated plasma beam of a reactive medium by a plasma discharge by supplying a working gas and moving the plasma beam over the at least one surface. The plasma beam is active in a chemical way such that a surface pre-treatment can be achieved in order to increase wettability of the at least one surface to be treated. On the other hand the temperature of the plasma beam is at such a low level that delicate surfaces are not damaged. Preferably, the plasma beam is provided by a plasma source comprising a plasma head. The plasma head may be a static plasma head such as PFW 10 or a rotatable plasma head such as RD 1004. The plasma source also comprises a plasma nozzle such as e.g. article-No.: 10147, 10148 or 15029, commercially available for example from the company Plasmatreat GmbH, Steinhagen, Germany.

Preferably, the working gas is an oxygen-carrying gas. Optionally, the working gas is air. This reduces costs as no inert atmosphere around the plasma beam has to be created.

In an embodiment of the invention the atmospheric plasma is a low temperature plasma, wherein the plasma beam has a temperature of at most 300°C in proximity to the cleaned at least one surface.

In an embodiment of the invention multiple plasma beams of higher reactive medium are collectively moved over the cleaned at least one surface. Thus, the area which can be treated at once is increased and the time needed to clean the at least one surface of the MDI container is reduced.

In another embodiment of the invention the at least one surface is an inner surface of the container. The inner surface is the surface which comes into contact with the drug formulation stored in the MDI-container. Preferably, the outer surface of the MDI container is not treated during the method of the invention.

In an embodiment the container comprises a material chosen from the group consisting of aluminum, aluminum alloy and stainless steel. The material may be chosen depending on the physical properties needed in the respective application, e.g. in order to withstand the corresponding pressure of the solution to be used with the MDI container.

In another embodiment the container is deep drawn from a metal blank or manufactured via impact extrusion. Preferably, the metal blank is deep drawn into the MDI container in multiple drawing steps by use of a deep drawing transfer press having multiple punches and dies.

The object of the present invention is also achieved by a processing line as defined in claim 15. The processing line is configured to process the method as defined above.

According to the invention the processing line is configured for cleaning and coating of at least one surface of a metered dose inhaler and comprises a cleaning station for cleaning the at least one surface with a cleaning solvent, an aqueous cleaning solution or an alcohol based cleaning solution. The processing line further comprises a plasma treatment station which is configured to subject the cleaned at least one surface through an atmospheric plasma treatment. The processing line further includes a coating station for coating the plasma treated at least one surface with a coating composition containing a flouropolymer resin or for coating the plasma treated at least one surface by a plasma coating treatment. Optionally, the plasma treatment is a low pressure plasma treatment.

Optionally, in case the plasma treated at least one surface is coated with a coating composition containing a flouropolymer resin the processing line may further comprise a cross-linking station. The cross-linking station may be configured to subject the coated at least one surface to a cross-linking step to provide an adherent continuous coating on the at least one surface of the container.

Optionally, in case the plasma treated at least one surface is coated by a plasma coating treatment no further cross-linking station is needed.

### BRIEF DESCRIPTION OF THE FIGURES

The invention will now be described in connection with an exemplary embodiment shown in the Figures in which:
- Figure 1: shows a process flow diagram of a known cleaning and coating method;
- Figure 2: shows a process flow diagram of the method according to the present invention and
- Figure 3: shows a schematically view of a processing line according to the present invention.

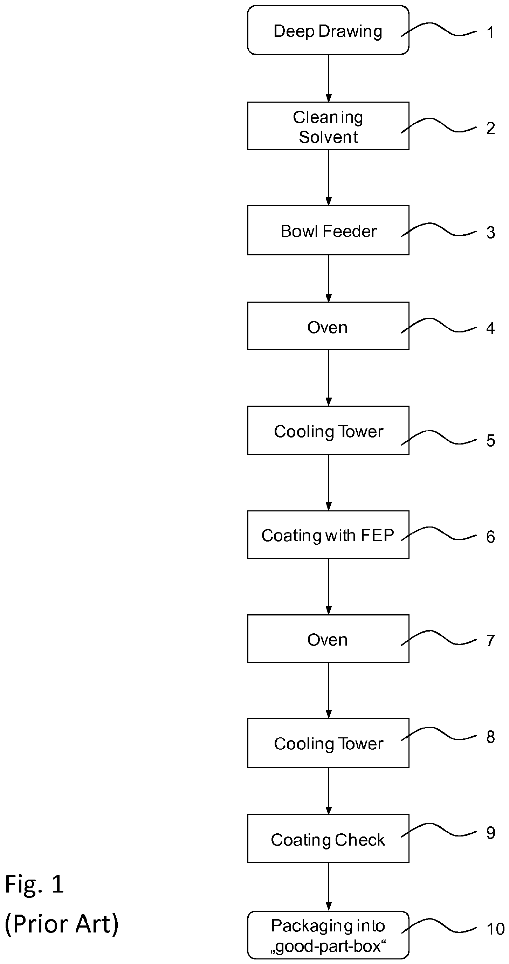

Figure 1 shows a known method of cleaning and coating at least one surface, in particular an inner surface, of a metered dose inhaler container.

In a first step 1 the MDI container is deep drawn, e.g. from a metal blank, or manufactured via impact extrusion.

In a second step 2 an inner surface of the container is cleaned with a cleaning solvent such as tretrachlorethene (PER). In particular the cleaning solvent is used in order to remove most of the hydrocarbon lubricants used for the production of the MDI container.

In a third step 3 the MDI container is fed into a bowl feeder in order to be conveyed one by one to subsequent processing stations.

In a fourth method step 4 the MDI container is inserted into an oven (not shown) in order to remove residual hydrocarbons. In the oven the MDI container is typically heated above 200°C. Subsequently, in a further method step 5 the MDI container is provided to a cooling tower (not shown) in order to cool the container down to room temperature.

In a further method step 6 the cleaned inner surface is subjected to a coating composition containing a flouropolymere resin for example fluorinated ethylene propylene (FEP).

In a further method step 7 the coated inner surface is subjected to a cross-linking step to provide an adherent continuous coating on the inner surface of the container. In particular the MDI container is moved into an oven and the MDI container is heated to a temperature that initiates chemical cross-linking of the coating. The temperature and time at temperature are selected based on measurements of coating cross-linking density and desorption of volatile organic compounds based on measurements with gas chromatography/mass spectrometry.

In a further method step 8 the MDI container is cooled in a cooling tower (not shown). Thereafter, in method step 9, the MDI container is checked in order to insure proper quality of the coating and subsequently in method step 10 packaged into a "good part box".

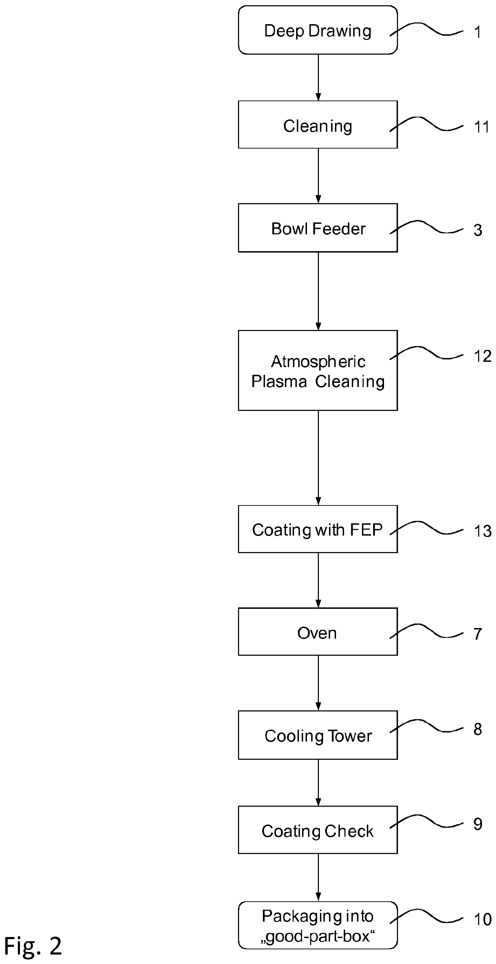

Figure 2 shows a method of cleaning and coating according to the present invention.

In a first method step 1 the MDI container is deep drawn from a metal blank in multiple deep drawing steps. Alternatively the container is manufactured via impact extrusion. The container comprises a material select from the group consisting of aluminum, aluminum alloy and stainless steel. In particular, aluminum is preferred.

In a second step 11 according to the invention the at least one surface, which is an inner surface of the container, is cleaned. Preferably, an outer surface of the MDI container is not treated at all during the method according to the present invention.

In the second method step 11 the inner surface of the MDI container is cleaned with a cleaning solvent, an aqueous cleaning solution or an alcohol based cleaning solution. All of the three mentioned cleaning solutions are used as alternatives. The cleaning solvent comprises chlorinated hydrocarbons, in particular tetrachlorethene (PER). The aqueous cleaning solution comprises an anionic surfactant and an emulsifier. The alcohol based cleaning solution is for example a succinic acid ester.

Preferably, all method steps of the method according to the present invention are carried out one after another at one single location. Optionally, the first two method steps (1 and 11) and the subsequent method steps are conducted at different locations, e.g. at different production plants.

In a third method step 3 the cleaned MDI container is fed into a bowl feeder in order to be conveyed one by one to subsequent processing stations to undergo further method steps.

In a subsequent method step 12 according to the invention the inner surface is subjected to an atmospheric plasma treatment. The atmospheric plasma treatment includes generating a concentrated plasma beam of a reactive medium by plasma discharge while supplying a working gas such as an oxygen-carrying gas, e.g. air, and moving the plasma beam over the at least one surface. The atmospheric plasma is a low temperature plasma, wherein the plasma beam has a temperature of at most 300°C in proximity to the cleaned inner surface. Preferably, multiple plasma beams of a reactive medium are collectively moved over the cleaned inner surface of the MDI-container.

In a subsequent method step 13 the plasma treated inner surface is subjected to a coating composition containing a fluoropolymer resin or alternatively to a plasma coating treatment. A method and corresponding device for a plasma coating treatment is for example disclosed in document EP 2 539 082 B1 to which reference is made in its entirety. Optionally, the plasma treatment is a low pressure plasma treatment.

The coating composition is a solvent based coating composition or alternatively an aqueous based coating composition. The solvent based coating composition comprises a solvent chosen from the group consisting of n-methyl-2-pyrolidon (NMP), n-butyl-2-pyrrolidone (NBP) and methyl isobutyl ketone (MIBK). The alternatively used aqueous based coating composition comprises 20 to 40wt% of the fluoropolymer resin with one or more alcohols as wetting agents and the balance water.

The fluoropolymer resin is chosen from the group consisting of: polytetrafluoroethylene (PTFE), fluorinated ethylene propylene (FEP) and perfluoralkoxy polymeres (PFA).

In a subsequent method step 7 the coated inner surface of the MDI container is subjected to a cross-linking step to provide an adherent continuous coating on the inner surface of the container. The MDI container is heated to a temperature that initiates chemical cross-linking of the coating.

The subsequent method steps 8 to 10 correspond to those as described with regard to the known method of cleaning and coating according to Figure 1.

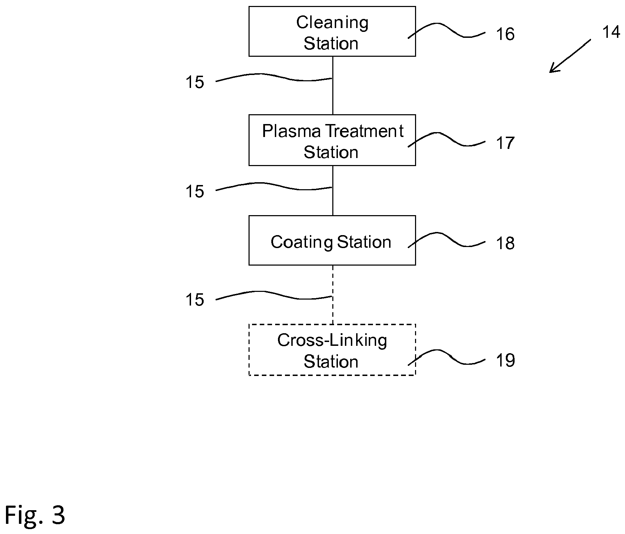

Figure 3 shows a processing line 14 for the coating of the inner surface of a MDI container (now shown) according to the invention. The processing line 14 comprises a conveyor 15 by use of which MDI container are conveyed, preferably on a tray (not shown), from one station of the processing line 14 to subsequent stations. The processing line 14 is configured to process the method according to the present invention as described above.

The processing line 14 comprises a cleaning station 16 for cleaning the inner surface of the MDI container with a cleaning solvent, an aqueous cleaning solution or an alcohol based cleaning solution.

The processing line 14 further comprises a plasma treatment station 17 which is configured to subject the cleaned inner surface to an atmospheric plasma treatment.

The processing line 14 further comprises a coating station 18 for coating the plasma treated inner surface with a coating composition containing a fluoropolymer resin or coating the plasma treated inner surface by plasma treatment. Preferably, the plasma treatment is a low pressure plasma treatment.

In case a coating composition containing a fluoropolymer is used the processing line 14 further comprises a cross-linking station 19 (shown in dotted lines). The cross-linking station 19 is configured to subject the coated inner surface to a cross-linking step to provide an adherent continuous coating on the inner surface of the container.

In case the inner surface of the container is coated by plasma treatment no additional cross-linking step is needed and the processing line 14 does not comprise a cross-linking station, accordingly.

## Claims

1. A method of cleaning and coating at least one surface of a metered dose inhaler container, the method comprising the steps of:
a) cleaning the at least one surface of the container with a cleaning solvent, an aqueous cleaning solution or an alcohol based cleaning solution;
b) subjecting the cleaned at least one surface to an atmospheric plasma treatment;
c) subjecting the plasma treated at least one surface to a coating composition containing a fluoropolymer resin or to a plasma coating treatment.

2. The method of claim 1, wherein the cleaning solvent comprises chlorinated hydrocarbons.

3. The method of any of the preceding claims, wherein the aqueous cleaning solution comprises:
a. an anionic surfactant; and
b. an emulsifier;

4. The method of any of the preceding claims, wherein the coating composition is solvent based or aqueous based.

5. The method of claim 5, wherein the solvent based coating composition comprises a solvent chosen from the group consisting of: n-methyl-2-pyrrolidone (NMP), n-butyl-2-pyrrolidone (NBP) and methylisobutylketone (MIBK).

6. The method of any of the preceding claims, wherein the aqueous based coating composition comprises 20 to 40 percent by weight of the fluoropolymer resin with one or more alcohols as wetting agents and balance water.

7. The method of any of the preceding claims, wherein the fluoropolymer resin is chosen from the group consisting of: polytetrafluoroethylene (PTFE), fluorinated ethylene propylene (FEP) and perfluoralkoxy polymeres (PFA).

8. The method of any of the preceding claims, wherein the atmospheric plasma treatment includes generating a concentrated plasma beam of a reactive medium by plasma discharge while supplying a working gas and moving the plasma beam over the at least one surface.

9. The method of claim 8, wherein the working gas is an oxygen-carrying gas.

10. The method of claim 8, wherein the atmospheric plasma is a low temperature plasma, wherein the plasma beam has a temperature of at most 300°C in proximity to the cleaned at least one surface.

11. The method of claim 6, wherein multiple plasma beams of a reactive medium are collectively moved over the cleaned at least one surface.

12. The method of any of the preceding claims, wherein the at least one surface is an inner surface of the container.

13. The method of any of the preceding claims, wherein the container comprises a material selected from the group consisting of aluminum, aluminum alloy and stainless steel.

14. The method of any of the preceding claims, wherein the container is deep drawn from a metal blank or manufactured via impact extrusion.

15. A processing line for cleaning and coating of at least one surface of a metered dose inhaler container, the processing line comprising:
a cleaning station (16) for cleaning the at least one surface with a cleaning solvent, an aqueous cleaning solution or an alcohol based cleaning solution,
a plasma treatment station (17) which is configured to subject the cleaned at least one surface to an atmospheric plasma treatment,
a coating station (18) for coating the plasma treated at least one surface with a coating composition containing a fluoropolymer resin or for coating the plasma treated at least one surface by a plasma coating treatment,
wherein the processing line is configured to process the method according to any of claims 1 to 14.
